# EUROPÄISCHE PATENTANMELDUNG

(11) **EP 0 621 043 A2**
(43) Veröffentlichungstag der Anmeldung: **26.10.1994**
(21) Anmeldenummer: 94102247.7
(22) Anmeldetag: 14.02.1994
(51) Int. Cl.: A61L 27/00, C03C 4/00, A61K 6/083, A61F 2/30, B29C 45/00, B28B 7/36

(54) **Verfahren zum Herstellen von Endoprothesen aus Glasionomerzement**

(30) Priorität: 22.03.1993 DE 4309212
(71) Anmelder: THERA Patent GmbH & Co. KG Gesellschaft für industrielle Schutzrechte, D-82229 Seefeld (DE)
(72) Erfinder: Herold, Wolf-Dietrich, Dr., D-82229 Seefeld (DE); Koran, Peter, Dr., D-82362 Weilheim (DE); Brandhorst, Gerd, D-86899 Landsberg (DE)
(74) Vertreter: Strehl Schübel-Hopf Groening & Partner

(57) **Zusammenfassung**

Endoprothesen lassen sich wirtschaftlich, formgenau und in einer an die Erfordernisse im medizinischen Bereich angepaßten Weise aus Glasionomerzement dadurch herstellen, daß man eine fließfähige Zementmasse herstellt, unter Druck in eine Formwerkzeug einbringt und darin aushärten läßt. Als Formwerkzeug wird eine dünnwandige, nicht selbst formstabile Schale verwendet, die beim Einpressen der Zementmasse von einem äußeren formstabilen Formwerkzeug gestützt wird. Die ausgehärtete Prothese kann bis zu ihrer Implantierung in der Schale belassen werden, die in diesem Fall gleichzeitig als Verpackung dient.

## Beschreibung

Aus EP-A-0 386 525 ist die Herstellung von Knochenersatzstrukturen aus Glasionomerzement bekannt. Dabei wird davon ausgegangen, daß der Operateur das Knochenersatzteil während der Operation aus frisch angemischtem Glasionomerzement manuell formt, es außerhalb des Körpers erhärten läßt und die ausgehärtete Prothese anschließend implantiert. Als Alternative ist die Möglichkeit beschrieben, einen aus Glasionomerzement industriell vorgefertigten, ausgehärteten Formkörper durch mechanische Bearbeitung den anatomischen Verhältnissen des jeweiligen Patienten anzupassen.

Im ersten Fall ist die genaue Formgebung dem Geschick des Operateurs überlassen. Bei zu ersetzenden größeren Knochenbereichen, etwa Teilen von Schädelknochen, ist eine manuelle Verformung der noch plastischen Zementmasse kaum möglich; bei solchen größeren Ersatzteilen läßt sich eine ausreichende Genauigkeit hinsichtlich Form und Größe kaum erreichen.

Der zweite Fall gestattet zwar an sich die Herstellung von Endoprothesen mit individueller Anpassung unter Anwendung einer numerisch gesteuerten Werkzeugmaschine, die das Implantat nach computertomographisch gewonnenen Daten aus einem Rohling erzeugt, wie dies aus EP-B-0 255 797 bekannt ist. Für eine solche Vorgehensweise haben sich Glasionomerzemente jedoch aus folgenden Gründen als problematisch erwiesen:
(a) Glasionomerzemente sind sprödharte Werkstoffe mit geringer Zugfestigkeit und daher für eine Fräsbearbeitung ungeeignet. Insbesondere grazile Formen lassen sich durch spanabhebende Bearbeitung kaum erzeugen.
(b) Die Oberfläche des Glasionomerzements muß im ausgehärteten Zustand ständig feucht gehalten werden. Trockenheit führt bereits nach wenigen Sekunden, ausgehend von der Oberfläche, zu Sprüngen im Material. Diese spezielle Eigenschaft macht die Bearbeitung auf herkömmlichen Fräsmaschinen problematisch, weil es praktisch unmöglich ist, während des Auf- und Abspannens sowie während des gesamten Fräsvorgangs alle Oberflächen des Rohlings feucht zu halten. Außerdem fördert die bei der Zerspanung des Materials erzeugte Wärme die Austrocknung und erhöht daher die Gefahr der Zerstörung des Materials.
(c) Endoprothesen sind zum Langzeit-Verbleib im Körper bestimmt und müssen daher absolut steril sein. Eine saubere, möglichst keimfreie Herstellung auf einer komplizierten Fünf-Achs-Fräsmaschine, die mit Kühlflüssigkeit arbeiten soll, ist - wenn überhaupt - nur mit hohem Aufwand zu realisieren. Eine derartige spanabhebende Bearbeitung ist prinzipiell schwer vereinbar mit den rechtlichen Auflagen für medizinische Produkte, gemäß denen bei der Herstellung eines Produktes die Sauberkeit während aufeinander folgender Verfahrensschritte zu steigern ist.
(d) Bei der mechanischen Bearbeitung des sprödharten Glasionomerzements entsteht feiner Staub, der sich an der Oberfläche des Implantats ablagert. Da solche Ablagerungen nach der Implantation eine Gefährdung des Organismus darstellen, ist zwischen der Herstellung und der Implantation eine Reinigungsprozedur unerläßlich.
(e) Beim Aushärten eines größeren Materialblocks (Rohlings) entstehen in dessen Inneren Materialspannungen. Wegen ihrer Sprödheit neigen Glasionomerzemente zur Bildung von Spannungsrissen, so daß auch nach der mechanischen Bearbeitung verhältnismäßig geringe Belastungen zur Zerstörung des Knochenersatzteils führen können.
(f) Aus Sicherheitsgründen sollten bei einer Individualprothese dem Chirurgen bei der Operation zwei gleiche Implantate zur Verfügung stehen. Dies führt wegen des aufwendigen Fräsvorgangs zu einer erheblichen Kostensteigerung.

Der Erfindung liegt die Aufgabe zugrunde, ein Verfahren anzugeben, mit dem sich Endoprothesen aus Glasionomerzement wirtschaftlicher, genauer und an die Erfordernisse eines operationsgerechten Betriebs besser angepaßten Weise herzustellen.

Die erfindungsgemäße Lösung dieser Aufgabe ist im Anspruch 1 gekennzeichnet.

Danach wird der Glasionomerzement aus Pulver und Flüssigkeit unter kontrollierten Umgebungsbedingungen in einer solchen Konsistenz angemischt, daß er sich unter Druck in ein teilbares Formwerkzeug einpressen läßt. Als Formwerkzeug wird dabei eine dünnwandige, nicht selbst formstabile Schale verwendet, die beim Einpressen der Zementmasse von einem äußeren formstabilen Formwerkzeug gestützt wird.

Das erfindungsgemäße Verfahren hat eine Reihe von Vorteilen:
(i) Die Schale des Formwerkzeugs kann aus einem beliebigen, leicht fräsbaren und preisgünstigen Material, insbesondere Kunststoff, hergestellt werden. Insbesondere für Standard-Implantate können auch spritzgegossene oder tiefgezogene Formen, zum Beispiel aus geeignetem Kunststoff, als Formwerkzeuge hergestellt werden. Gleichzeitig wird der Einsatz von Glasionomerzement auf ein Minimum beschränkt, und die Herstellung von zwei oder mehreren identischen Implantaten ist ohne großen Aufwand möglich.
(ii) Wegen des Einsatzes von preisgünstigem Material für die Schale ist das Verfahren auch bei Einmalgebrauch (für Individualprothesen) wirtschaftlich.
(iii) Da die Schale die Endoprothese vollständig umschließt, wird ein Austrocknen der Oberfläche während der Aushärtung mit Sicherheit vermieden.
(iv) Eine keimfreie Herstellung von Prothesen läßt sich dadurch erreichen, daß die Schale gereinigt und sterilisiert wird und die Herstellung der Zementmasse sowie das Einpressen der Masse in die Schale unter hygienisch kontrollierten Bedingungen erfolgen.
(v) Beim Einpressen des Glasionomerzements in eine der endgültigen Form entsprechend erzeugte Formwerkzeugschale entfällt jede Art der mechanischen Nachbearbeitung. Das fertige Implantat weist daher eine geschlossene, saubere und von anhaftenden Materialpartikeln freie Oberfläche auf.
(vi) Wegen der geschlossenen Oberfläche ist auch die Gefahr von Spannungsrissen geringer.

Vorteilhafterweise wird die Schale selbst unter Verwendung des äußeren Formwerkzeugs hergestellt.

Wird das Formwerkzeug nach computertomographisch gewonnenen Daten mittels einer numerisch gesteuerten Werkzeugmaschine direkt hergestellt, so lassen sich auch große Implantate mit hoher Genauigkeit in Übereinstimmung mit den anatomischen Verhältnissen des betreffenden Patienten herstellen.

Die ausgehärtete Prothese kann bis zu ihrer Implantierung in der als Verpackung dienenden Schale belassen werden. In diesem Fall bleibt das Knochenersatzteil bis zu seinem endgültigen Einsatz von der umschließenden Schale gestützt, gegebenenfalls steril sowie gegen Austrocknung geschützt.

Vorteilhafterweise wird eine flüssigkeits-undurchlässige Schale oder ein solche, die keine Flüssigkeitsaufnahme aufweist, verwendet, oder das Material der Schale wird mit Flüssigkeit vorkonditioniert, um einem Austrocknen der Implantat-Oberfläche während und nach dem Aushärtvorgang weiter entgegenzuwirken. Eine Vorkonditionierung des Materials der Schale mit Flüssigkeit, etwa Wasser, ist insbesondere dann zweckmäßig, wenn ein Kunststoff mit hoher Wasseraufnahme, zum Beispiel transparentes, gammasterilisierbares Polyamid, verwendet wird.

Wird ein Formwerkzeug aus transparentem Material verwendet, so ergibt sich der Vorteil, daß eine Blasenbildung in dem Implantat bereits vor dem zeitaufwendigen Aushärtungsprozeß erkennbar wird und systematische Fehler beim Einpressen der Masse in das Formwerkzeug erkannt und abgestellt werden können.

Dabei sind Kunststoffe als Material für die Schale des Formwerkzeugs besonders geeignet, weil sie im Gegensatz zu Metallen keine hohen Adhäsionskräfte gegenüber ausgehärtetem Glasionomerzement aufweisen.

## Patentansprüche

1. Verfahren zum Herstellen von Endoprothesen aus Glasionomerzement, wobei eine fließfähige Zementmasse hergestellt und durch plastische Verformung auf die gewünschte Gestalt der Prothese gebracht wird,
dadurch gekennzeichnet,
daß die fließfähige Zementmasse unter Druck in ein Formwerkzeug eingebracht wird und darin aushärtet, und
daß als Formwerkzeug eine dünnwandige, nicht selbst formstabile Schale verwendet wird, die beim Einpressen der Zementmasse von einem äußeren formstabilen Formwerkzeug gestützt wird.

2. Verfahren nach Anspruch 1, wobei die Schale selbst unter Verwendung des äußeren Formwerkzeugs hergestellt wird.

3. Verfahren nach Anspruch 1 oder 2, wobei das Formwerkzeug nach computertomographisch gewonnenen Daten mittels einer numerisch gesteuerten Werkzeugmaschine direkt hergestellt wird.

4. Verfahren nach einem der Ansprüche 1 bis 3, wobei die ausgehärtete Prothese bis zu ihrer Implantierung in der als Verpackung dienenden Schale belassen wird.

5. Verfahren nach einem der Ansprüche 1 bis 4, wobei eine flüssigkeits-undurchlässige Schale verwendet wird.

6. Verfahren nach einem der Ansprüche 1 bis 5, wobei eine Schale verwendet wird, die keine Flüssigkeitsaufnahme aufweist.

7. Verfahren nach Anspruch 1, daß das Material der Schale mit Flüssigkeit vorkonditioniert wird.

8. Verfahren nach einem der Ansprüche 1 bis 7, wobei eine Schale aus sterilisierbarem Material verwendet wird.

9. Verfahren nach einem der Ansprüche 1 bis 8, wobei ein Formwerkzeug aus transparentem Material verwendet wird.

10. Verfahren nach einem der Ansprüche 1 bis 9, wobei eine Schale aus Kunststoff verwendet wird.
